# EUROPEAN PATENT SPECIFICATION

(11) **EP 3 552 541 B1**
(45) Date of publication and mention of the grant of the patent: **29.01.2025**
(21) Application number: 19168794.6
(22) Date of filing: 11.04.2019
(51) Int. Cl.: A61B 5/00, A61B 5/291

(54) **SYSTEM FOR THE DETECTION OF ELECTROENCEPHALOGRAPHIC SIGNALS**
SYSTEM ZUM MESSUNG ELEKTROENZEPHALOGRAPHISCHER SIGNALE
SYSTÈME DE DÉTECTION DE SIGNAUX ÉLECTRO-ENCÉPHALOGRAPHIQUES

(30) Priority: 12.04.2018 IT 201800004455
(43) Date of publication of application: 16.10.2019
(73) Proprietor: SPES MEDICA S.P.A., 16153 Genova (GE) (IT)
(72) Inventor: Mistretta, Matteo, 16148 Genova (IT); Cotella, Elena, 16031 Bogliasco (IT); Phelan, Patrick, Newburyport, MA 01950 (US)
(74) Representative: Bottino, Giovanni

(56) References cited:
- EP-B1- 0 934 593
- WO-A1-2012/026638
- WO-A1-2014/072582
- GB-A- 1 299 449
- KR-B1- 101 037 405
- US-A- 5 540 033
- US-A- 5 800 685
- US-A1- 2007 106 170
- US-A1- 2009 105 576
- US-A1- 2013 131 746
- US-A1- 2016 228 060
- US-B2- 11 375 952
- NIKULIN V V ET AL: "Miniaturized electroencephalographic scalp electrode for optimal wearing comfort", CLINICAL NEUROPHYSIOLOGY, ELSEVIER, AMSTERDAM, NL, vol. 121, no. 7, 1 July 2010 (2010-07-01), pages 1007 - 1014, XP027064564, ISSN: 1388-2457, [retrieved on 20100315], DOI: 10.1016/J.CLINPH.2010.02.008
- NEUMAN M R ED - BRONZINO JOSEPH D: "Chapter 48: Biopotential Electrodes", 1 January 2000, THE BIOMEDICAL ENGINEERING HANDBOOK; [THE ELECTRICAL ENGINEERING HANDBOOK SERIES], BOCA RATON, FLA. [U.A.] : CRC PRESS [U.A.], US, PAGE(S) 1 - 12, ISBN: 978-3-540-66351-5, XP002566008

## Description

The present invention relates to a system for detecting electroencephalographic signals comprising at least one electrode for transmission of the detected signal, conduction means of said signal interposed between the electrode and the scalp of a patient and fixation means of said electrode to the scalp.

What was just described is the common configuration of systems commonly used for the detection and recording of the electroencephalographic signals (EEG).

The recording of EEG signals is a non-invasive practice, involves the positioning of one or more electrodes in contact with the scalp of a patient through an electrolyte, for transmitting the signal to a processing unit.

The signal is detected by the electrode thanks to the presence of a layer of conductive gel interposed between the electrode and the scalp.

In view of the nature of the detected signal, it is evident that a correct registration and subsequent processing of EEG signals require a firm positioning of the electrode so as to provide the processing unit with accurate data to avoid the generation of artifacts, or inaccurate readings that generate an incorrect analysis.

To ensure a correct positioning of the electrode, the systems known in the state of the art use glues, creams, etc. which give a firm fixation in the desired position.

One of the major disadvantages in the use of these glues, creams, etc. is related to the cleaning of the scalp of the patient after their use: in fact, the removal of glues, creams, etc. is not particularly effortless, easy and atraumatic and requires a careful cleaning.

In systems known in the state of the art, the cleaning procedure on the scalp is necessarily carried out with detergent materials and/or solvents, not always suited to the scalp.

In fact, the used solvents must be particularly aggressive in order to obtain a satisfactory cleaning the scalp which allows the removal of the used glues and creams.

These effects are particularly troublesome for the patient if, as happens in common practice, the recording of EEG signals requires the use of numerous electrodes, arranged along the entire surface of the skull of the patient.

Therefore, in systems known in the state of the art, the procedure for cleaning and removing the electrodes has no negative aspects which are just related to the harshness of the used solvents, but also disadvantages in terms of timing.

These disadvantages are found not only in the cleaning process but also in the part preceding the examination and preparation of the patient, i.e. the positioning of the electrodes.

In fact the use of conductive gel in combination with glues for the positioning of the electrodes requires a long duration positioning procedure.

Some systems known in the state of the art seek to avoid the use of glues through the positioning of the electrodes supported by elastic material.

However, these electrodes require, in addition to the use of elastic caps that cover the skull of the patient, in order to fix the electrode in position, also the use of liquid gel for the contact with the scalp and the creation of the registration interface.

It is evident that this solution, in addition to increasing the structural complexity of the whole system, requires the availability of owning a range of different sizes of caps that can cover as much as possible the different patients and set-up requires a particularly long configuration time of the entire system.

Therefore, there is a need, not satisfied by the systems known in the state of the art, to provide a detection system of EEG signals which overcomes the disadvantages described above, ensuring a simple and functional positioning of the electrodes. KR 101 037 405 B1 discloses a cup electrode. GB 1 299 449 A discloses pre-packaged hydrogels.

The present invention, defined by a system according to claim 1, a method according to claim 5 and a method according to claim 7 (with further embodiments found in the respective dependent claims 2-4, 6), achieves the above objects by providing a system as described above, in which the conduction means and the fixation means are constituted by a single hydrogel layer.

A particularly dense, nearly solid polymer is made, and has both conductive and adhesive features, so as to speed up the electrode positioning process, using a single component.

The hydrogel may for example be provided in the form of a drop/tablet contained in a dispenser/blister pack capable of allowing a user to use and position the hydrogel on the scalp of the patient in points of interest through the use of a cup electrode.

According to the invention, the hydrogel material is provided in the form of a drop/tablet element.

According to this configuration, the hydrogel is therefore pre-dosed, i.e. provided in predetermined doses, sufficient to conduct the signal and to ensure a sufficiently wide surface for gluing.

The particularly dense nature of the hydrogel allows to create finite elements that can be taken one by one to be positioned and removed easily from the scalp of the patient.

It is specified that some embodiments illustrated in the following will describe a specific embodiment of the shape of the drop/tablet element.

The removal of these drop/tablet element leaves minimal residue on the hair of the patient, easily removable with aqueous solutions, not harmful to the scalp of the patient.

The shape of the drop/tablet element also has a specific advantage, i.e. to provide a sort of bearing interposed between the scalp and the electrode, preventing the trauma due to the pressure of the electrode on the scalp, effect known with the name of "skin-breakdown".

For this reason, the hydrogel that constitutes the drop/tablet element must have a density such as to be sufficiently rigid for the creation of finite elements, but at the same time sufficiently soft, i.e. mechanically yielding, to change its surfaces so as to adapt them to the contact surfaces of the skull of the patient and/or the electrode, once the drop/tablet element is used for the detection of the signals.

Moreover, the hydrogel that constitutes the drop/tablet element also has both conductive and adhesive features with respect to the skull of the patient.

The system object of the present invention can involve any type of cup electrode known in the state of the art, which has a surface facing the scalp adapted to house said drop/tablet element.

The shape of the drop/tablet element is particularly advantageous in combination with the cup electrodes, especially if the drop/tablet element has a size such as to be housed inside the electrode cup.

This configuration has advantageous effects also on the positioning of the electrodes: the cup electrode can be used by a user in a similar manner to a spoon, which collects the drop/tablet element to then be immediately placed on the scalp of the patient.

The above mentioned advantages are particularly relevant in the case in which a plurality of electrodes and a plurality of drop/tablet elements are provided.

Moreover in order to facilitate the collection of such drop/tablet elements by the cup electrodes, it is according to the invention to group all the drop/tablet element through a single support element, being a plate-like support element, on which the drop/tablet elements are placed.

The hydrogel elements remain attached to the upper surface of the plate-like element and can be removed from the electrode cup, to ensure their positioning on the scalp of the patient.

Since the electrode has a wire for the connection of the processing unit, in order to prevent accidental tractions of the operator with the wire from moving the electrode, an embodiment variant of the system according to the present invention provides retention means for retaining the electrode in position.

For example, such retention means can be constituted by an adhesive support, placed in contact with the scalp of the patient and the outer surface of the head of the electrode.

In addition, in the light of the features described above, the present disclosure relates to an example by itself not covered by the claims of a disposable electrode for detecting electroencephalographic signals that comprises a cup electrode and a hydrogel drop/tablet element inserted inside the cup of said electrode.

It is therefore possible to provide a single pack of a disposable electrode already ready to be positioned on the scalp of a patient.

As for the embodiment variants described above, also in this case it is possible to provide an adhesive support for retaining the electrode in position on the scalp of the patient.

In the light of the advantageous features described heretofore, the present invention also relates to a method for the detection of electroencephalographic signals.

This method provides for the use of the system or of the electrode described above and in comprises the fixation of at least one electrode to the scalp of a patient for the transmission of the detected electrical signals.

According to the method object of the present invention, the fixation of the electrode includes the following steps:
a) positioning a drop/tablet element in contact with the electrode,
b) bonding of the electrode to the scalp of the patient through the drop/tablet element,
c) detection of electroencephalographic signals through the drop/tablet element.

Also in this case, the drop/tablet element allows the use of a single component that allows at the same time the conduction of EEG signals, as well as the gluing of the electrode to the head of the patient.

The application of the electrode is therefore immediate, fast, efficient and particularly "clean".

In combination with this embodiment, preferably the step of positioning of the drop/tablet element in contact with the electrode, provides for the collection of the drop/tablet element from the plate-like element and the insertion on said electrode, the electrode consisting of a cup electrode.

To improve the retention in position of the electrode, the step of gluing of the electrode provides a step of retaining the position of said electrode to the scalp of the patient through the use of a preformed adhesive support.

In conclusion, an object of the present invention is a method for creating the conduction and fixation means of the system described above according to claim 7.

Finally, it is specified that a further example by itself not covered by the claims also relates to a collection of hydrogel elements.

This collection includes hydrogel drop/tablet elements created according to one or more of the features described above and which are arranged on a plate-like element, in a similar manner to as some types of tablets or pills are inserted inside pharmaceutical blister packs, as will be illustrated later.

These and other features and advantages of the present invention will become clearer from the following description of some exemplary embodiments illustrated in the attached drawings in which:
Figures 1a and 1b show two views of the system object of the present invention according to a possible embodiment;
Figure 2 shows a sectional view of a tablet element coupled to an electrode belonging to the system object of the present invention;
Figure 3 is a block diagram showing the principle of the method object of the present invention.

It is specified that the figures attached to the present patent application illustrate some embodiments of the system and the method object of the present invention, in order to better understand its advantages and the described features.

These embodiments are therefore to be understood as purely illustrative and do not limit the scope of the inventive concept of the present invention, i.e. creating a system for detecting electroencephalographic signals that involves the fixation of at least one electrode to the scalp of a patient through a single layer of hydrogel, which guarantees both the gluing to the scalp and the transmission of electrical signals from the scalp to the electrode.

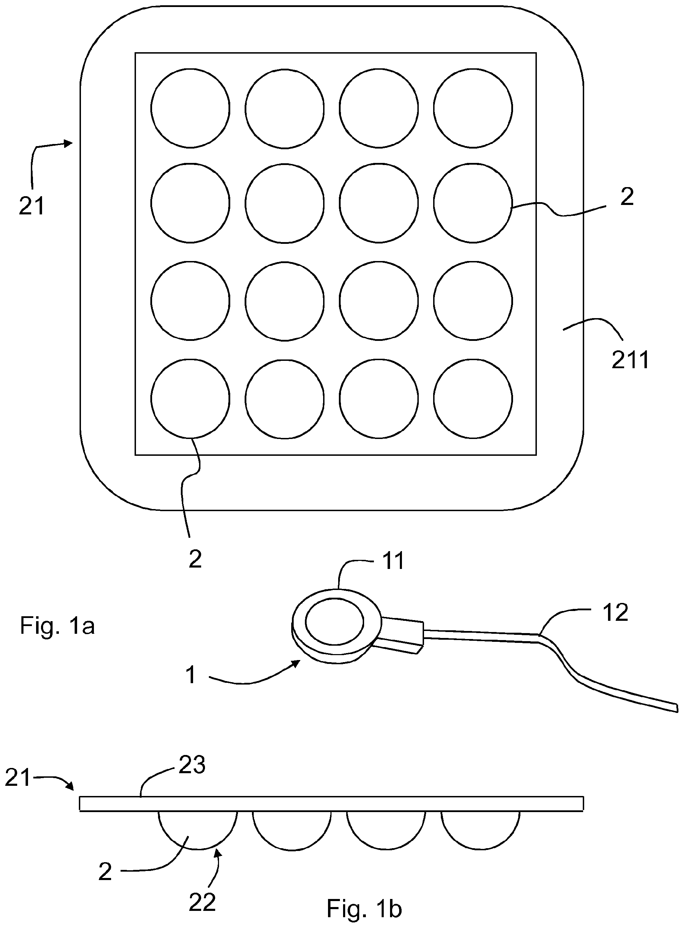

In particular, figures 1a and 1b illustrate a preferred embodiment of the system according to the present invention.

According to this embodiment, the system comprises at least one electrode 1 for transmitting the detected signal and conduction means of the signal interposed between the electrode 1 and the scalp of a patient, as well as fixation means of the electrode to the scalp.

In the variant illustrated in Figures 1a and 1b, the fixation means and the conduction means are constituted by a single drop/tablet element 2, made with a material of the hydrogel type.

Figure 1a shows a plurality of drop/tablet elements 2, while showing a single electrode 1.

It is evident that the system which is the object of the present invention can provide any number of electrodes 1 and any number of drop/tablet elements 2.

The electrode 1 is advantageously constituted by a cup electrode and has a cup end 11 and a signal transmission cable 12.

The cup unit 11 is adapted to be positioned on the skull of the patient, while the transmission cable 12 is responsible for the transmission of the detected signal to a processing unit, including inside electrocardiographs known in the state of the art.

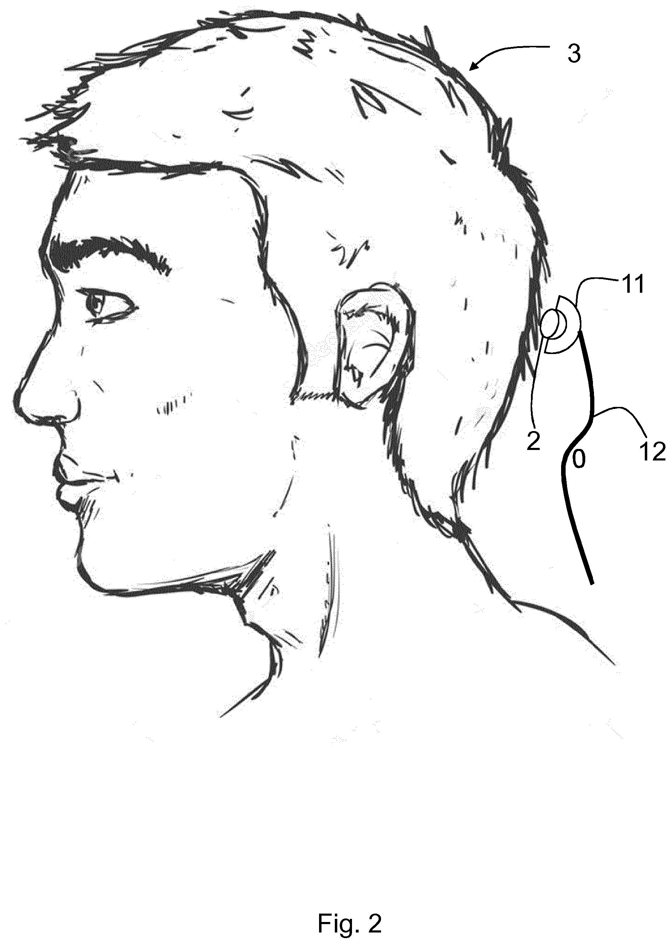

The cup end 11 constitutes a housing seat for the drop/tablet element 2, as illustrated in Figure 2.

Figure 2 shows a patient 3 and a section of the electrode 1 in combination with the drop/tablet element 2.

The cup end 11 houses in its interior the drop/tablet element 2.

For simplicity of illustration, the cross-section of the drop/tablet element 2 of Figure 2 corresponds to an ellipse, however, being constituted by a hydrogel, the drop/tablet element 2 is deformed in such a way as to adhere to the inner surface of the cup end 11 and to the scalp of the patient 3.

This deformation allows to create a seamless contact between the scalp of the patient 3 and the cup end 11 of the electrode 1.

Advantageously, the drop/tablet element 2 has dimensions such as to be housed inside the cup end 11, without that the excessive deformation of the drop/tablet element 2 causes an excessive spillage of hydrogel from the edges of the cup ends 11.

Advantageously, the dimensions of the drop/tablet element 2 are such as to allow it to be housed inside the cup end 11 and cover the edges facing toward the scalp.

In the condition of the positioning of the electrode 1, the drop/tablet element 2 therefore deforms its surfaces so as to create surfaces complementary to the scalp of the patient 3 and at the cup end 11.

According to a further embodiment, it is possible to provide retention means for retaining the electrode 1 in position, not shown in Figure 2, to assist the gluing action performed by the hydrogel of the electrode 1 to the scalp of the patient 3.

Such retention means are preferably constituted by an adhesive support, which can be positioned on the outer surface of the cup end 11 and with its ends fixed to the scalp of the patient 3.

According to the variant illustrated in Figures 1a and 1b, the system further comprises a plate-like support element 21 on which to place the plurality of drop/tablet elements 2.

The drop/tablet element 2 are none other than drops of hydrogel arranged on the upper surface of the plate-like element 21, made in the form of a sheet, preferably of plastic material, on which the drop/tablet elements 2 remain fixed thanks to their adhesive feature, until they are removed to be used in combination with the electrodes 1.

It is evident that the plate-like support element can be constituted by a simple sheet of plastic material on which drop/tablet elements 2 can be arranged.

According to the variant illustrated in Figures 1a and 1b, the plate-like element is constituted by a blister pack-type element 21 that presents housing seats 22 of the drop/tablet element 2.

The drop/tablet element 2 are deposited inside the housing seats 22, after which the blister pack 21 is closed by a protective plastic film 23.

The protective plastic film 23 is attached to the blister pack 21 in such a way that, once removed, the drop/tablet elements 2 remain inside the housing seats 22.

For example it is possible to provide a zone 211 in which the protective film 23 may be glued or heat-welded to the blister pack 21.

In alternative, it is possible to envisage that the blister packs 21 is constituted by a flat plate-like element without housing seats, on which the individual drop/tablet element are arranged.

The deposition of the hydrogel drop/tablet element 2 within the housing seats 22 confers to the elements a semicircle shape, adapted to be housed inside the cup end 11 of the electrode 1.

In a similar manner it is possible to provide a disposable electrode, preferably a cup electrode, in which there is a drop/tablet element 2 inserted inside the cup end 11.

The cup end 11 can for example be sealed through the use of a protective film in a manner quite similar to the protective film 23 described previously, in this case fixed to the edges of the cup end 11.

With particular reference to Figures 1a and 1b, the blister pack 21 may have a square shape, with side equal to 140 millimetres.

The housing seats 22, constituted by semicircular elements, have a diameter comprised between 20 and 22 millimetres and are mutually spaced by a measure between 3 and 5 millimetres.

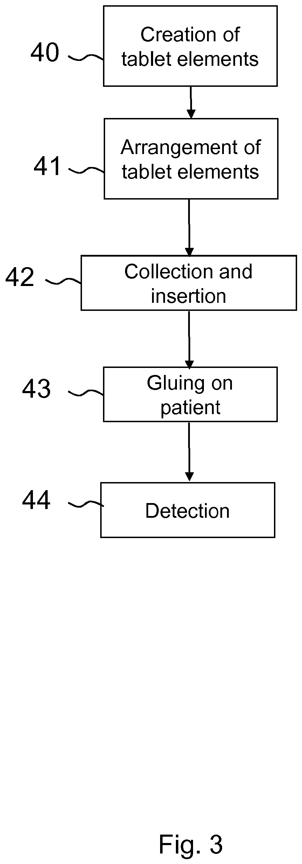

The system just described may be used to perform the method for detecting electroencephalographic signals object of the present invention, referred to a possible embodiment is illustrated in Figure 3 through a flow chart.

As in the methods known to the state of the art, the method object of the present invention involves the fixation of at least one electrode to the scalp of a patient for the transmission of the detected electrical signals.

From a general point of view, the method object of the present invention includes the following steps:
a) positioning a drop/tablet element in contact with the electrode,
b) bonding of the electrode to the scalp of the patient through the drop/tablet element,
c) detection of electroencephalographic signals through the drop/tablet element.

As anticipated, the tablet element 2 performs the dual function of gluing of the electrode and conducting the signal.

According to the variant shown in Figure 3, and on the basis of the system of Figures 1a, 1b and 2, the method object of the present invention can be preferably used with a plurality of electrodes, in order to place them on a wide surface of the skull of the patient to obtain the tracking of the EEG signal.

In particular, the fixation of the electrode includes the following steps:
- collection and insertion of each drop/tablet element on a corresponding electrode 1, step 42,
- gluing of the electrode 1 to the scalp of the patient 3, step 43,
- detection of the signal, indicated with 44.

The step indicated with 42 is preferably provided in combination with the use of the cup electrode 1.

Thanks to the fact they are made of adhesive hydrogel, the drop/tablet elements 2 are fixed to the plate-like element 21 in a stable manner.

When an operator must secure the electrode 1 to the scalp of a patient, thanks to the peculiar shape of the cup electrode 1, the operator will not have to handle the drop/tablet element 2, but it will be sufficient to use the cup end 11 as a spoon to remove the drop/tablet element 2 from the plate-like element or from the blister pack 21 and insert it to make contact with the inner surface of the cup end 11.

Once the coupling between the tablet element 2 and the electrode 1 is obtained, the operator positions the electrode 1 in the region of interest of the skull of the patient 3 and with a slight pressure, which causes the deformation of the drop/tablet element 2, glues the electrode 1 to the scalp of the patient 3.

At this point it is possible to detect the electroencephalographic signals to perform the examination of interest.

As is known in the state of the art, it is possible to provide at the beginning of the method, a step relative to the preparation of the skin of the patient, such as for example cleaning of the area concerned to the positioning of the electrode.

Finally, it is specified that the step 43 may provide a further fixation of the electrode 1 to the scalp of the patient 3, through the use of adhesive support, as previously described.

In particular, Figure 3 illustrates a possible embodiment which provides a method of use of the system object of the present invention and a method for the creation of the system object of the present invention.

The method of use is the one just described, in which an operator uses the packaged element constituted by the drop/tablet elements, inserted inside the blister packs of Figure 1b, places the drop/tablet elements on the electrode and carries out the examination.

This "operator-side" methodology, defined by steps 42 to 44, may be provided in combination with the method for the creation of the fixation and conduction means belonging to the system object of the present invention.

With reference to Figure 3, this method includes the following steps:
- creation of a plurality of hydrogel drop/tablet elements 2, indicated with 40,
- arrangement of the drop/tablet elements 2 on a plate-like support element, or inside the housing seats 22 of the blister pack 21, indicated with 41.

The steps from 40 to 44 may be provided in combination, or it is possible to provide that the steps 40 and 41 are performed by a producer of hydrogel or the like, while steps 42 to 44 can be performed by the users, i.e. by operators who intend to perform EEG signal detection analysis.

While the invention can be changed according to different modifications and alternative constructions, some preferred embodiments were shown in the drawings and described in detail.

It should be understood, however, that there is no intention of limiting the invention to the specific illustrated embodiment but, on the contrary, it aims at covering all modifications, alternative constructions, and equivalents falling within the scope of the invention as defined in the claims.

The use of "for example", "etc.", "or" refers to non-exclusive non-limiting alternatives, unless otherwise stated.

The use of "includes" means "includes but not limited to", unless otherwise stated.

## Claims

1. System for detecting electroencephalographic signals comprising at least one electrode (1) for the transmission of the detected signal, conduction means of said signal configured to be interposed between the electrode (1) and the scalp of a patient (3) and fixation means of said electrode (1) to the scalp,
said conduction means and said fixation means being constituted by a single layer of hydrogel,
said single layer of hydrogel is constituted by a finite element in the form of a drop or tablet element (2), provided in predetermined doses sufficient to conduct the signal and to ensure a sufficient wide surface for gluing, the particularly dense nature of the hydrogel allowing to create finite elements configured to be positioned and to be easily removed from the scalp of the patient the drop or tablet element (2) having a density such as to be sufficiently rigid for the creation of finite elements and at the same time sufficiently soft, i.e. mechanically yielding, to change its surfaces so as to adapt them to the contact surfaces of the skull of the patient and/or of the electrode,
the drop or tablet elements (2) being arranged as drops of hydrogel on the upper surface of a plate-like element, made in the form of a sheet, on which the drop or tablet elements (2) remain fixed thanks to their adhesive feature, until they are removed to be used in combination with the electrodes,
wherein said electrode is a cup electrode.

2. System according to claim 1, wherein said drop or tablet element (2) has a size such as to be housed on the electrode surface facing toward the scalp.

3. System according to one or more of the preceding claims, wherein retention means are provided for retaining the electrode (1) in position on the scalp of the patient (3).

4. System according to claim 3, wherein said retention means consist of an adhesive support.

5. Method for detecting encephalographic signals which involves the use of the system according to one or more of claims 1 to 4, which method comprises the fixation of the at least one electrode (1) to the scalp of a patient (3) for the transmission of the detected electrical signals, wherein said fixation involves the following steps:
a) positioning the drop or tablet element (2) in contact with said electrode (1),
b) bonding said electrode (1) to the scalp of the patient (3) through said drop or tablet element (2),
c) detection of the electroencephalographic signals through said drop or tablet element (2).

6. Method according to claim 5, wherein step b) includes a step of retaining said electrodes (1) in position on the scalp of the patient (3) through the use of an adhesive support.

7. Method for the creation of the conduction and fixation means of the system according to claims 1 to 4, wherein the method involves the following steps:
- creation of the plurality of hydrogel drop or tablet elements (2) according to claim 1,
- arrangement of said plurality of drop or tablet elements (2) as drops of hydrogel on the upper surface of the plate-like element (21 according to claim 1.

## Patentansprüche

1. System zum Erfassen von elektroenzephalographischen Signalen, umfassend mindestens eine Elektrode (1) für die Übertragung des erfassten Signals, Leitungsmittel des Signals, das dazu konfiguriert ist, zwischen der Elektrode (1) und der Kopfhaut eines Patienten (3) angeordnet zu sein, und Befestigungsmittel der Elektrode (1) an der Kopfhaut,
wobei das Leitungsmittel und das Befestigungsmittel aus einer einzelnen Schicht aus Hydrogel bestehen,
wobei die einzelne Schicht aus Hydrogel aus einem endlichen Element in Form eines Tropfen- oder Tablettenelements (2) besteht, das in vorbestimmten Dosen bereitgestellt wird, die ausreichen, um das Signal zu leiten und eine ausreichend breite Klebefläche zu gewährleisten, wobei die besonders dichte Beschaffenheit des Hydrogels es ermöglicht, endliche Elemente zu erzeugen, die dazu konfiguriert sind, auf der Kopfhaut des Patienten positioniert und leicht von dieser entfernt zu werden, wobei das Tropfen- oder Tablettenelement (2) eine solche Dichte aufweist, dass es ausreichend steif für die Bildung endlicher Elemente und gleichzeitig ausreichend weich, d. h. mechanisch nachgiebig, ist, um seine Oberflächen so zu verändern, dass sie sich an die Kontaktflächen des Schädels des Patienten und/oder der Elektrode anpassen,
wobei die Tropfen- oder Tablettenelemente (2) als Tropfen aus Hydrogel auf der oberen Oberfläche eines plattenartigen Elements angeordnet sind, das in Form einer Folie hergestellt ist, auf dem die Tropfen- oder Tablettenelemente (2) dank ihrer Klebeeigenschaft befestigt bleiben, bis sie für die Verwendung in Kombination mit den Elektroden entfernt werden,
wobei die Elektrode eine Napfelektrode ist.

2. System nach Anspruch 1, wobei das Tropfen- oder Tablettenelement (2) eine solche Größe aufweist, dass es auf der Elektrodenoberfläche, die der Kopfhaut zugewandt ist, aufgenommen werden kann.

3. System nach einem oder mehreren der vorstehenden Ansprüche, wobei Rückhaltemittel bereitgestellt werden, um die Elektrode (1) auf der Kopfhaut des Patienten (3) in Position zu halten.

4. System nach Anspruch 3, wobei die Rückhaltemittel aus einem Klebstoffträger bestehen.

5. Verfahren zum Erfassen von encephalographischen Signalen, das die Verwendung des Systems nach einem oder mehreren der Ansprüche 1 bis 4 beinhaltet, wobei das Verfahren die Befestigung der mindestens einen Elektrode (1) an der Kopfhaut eines Patienten (3) für die Übertragung der erfassten elektrischen Signale umfasst,
wobei die Befestigung die folgenden Schritte beinhaltet:
a) Positionieren des Tropfen- oder Tablettenelements (2) in Kontakt mit der Elektrode (1),
b) Verbinden der Elektrode (1) mit der Kopfhaut des Patienten (3) über das Tropfen- oder Tablettenelement (2),
c) Erfassen der elektroenzephalographischen Signale über das Tropfen- oder Tablettenelement (2).

6. Verfahren nach Anspruch 5, wobei Schritt b) einen Schritt des Haltens der Elektroden (1) in Position auf der Kopfhaut des Patienten (3) unter Verwendung eines Klebstoffträgers enthält.

7. Verfahren für die Bildung der Leitungs- und Befestigungsmittel des Systems nach den Ansprüchen 1 bis 4,
wobei das Verfahren die folgenden Schritte beinhaltet:
- Bildung der Vielzahl von Tropfen- oder Tablettenelementen (2) aus Hydrogel nach Anspruch 1,
- Anordnung der Vielzahl von Tropfen- oder Tablettenelementen (2) als Tropfen aus Hydrogel auf der oberen Oberfläche des plattenartigen Elements (21) nach Anspruch 1.

## Revendications

1. Système de détection de signaux électroencéphalographiques comprenant au moins une électrode (1) pour la transmission du signal détecté, des moyens de conduction dudit signal configurés pour être interposés entre l'électrode (1) et le cuir chevelu d'un patient (3) et des moyens de fixation de ladite électrode (1) au cuir chevelu,
lesdits moyens de conduction et lesdits moyens de fixation étant constitués par une couche unique d'hydrogel,
ladite couche unique d'hydrogel est constituée d'un élément fini sous la forme d'un élément de goutte ou de comprimé (2), fourni à des doses prédéterminées suffisantes pour conduire le signal et pour assurer une surface suffisamment large pour le collage, la nature particulièrement dense de l'hydrogel permettant de créer des éléments finis configurés pour être positionnés et facilement retirés du cuir chevelu du patient, l'élément de goutte ou de comprimé (2) ayant une densité telle qu'elle soit suffisamment rigide pour la création d'éléments finis et en même temps suffisamment molle, c'est-à-dire mécaniquement souple, pour changer ses surfaces de manière à les adapter aux surfaces de contact du crâne du patient et/ou de l'électrode,
les éléments de goutte ou de comprimé (2) étant disposés en gouttes d'hydrogel sur la surface supérieure d'un élément en forme de plaque, réalisé sous la forme d'une feuille, sur lequel les éléments de goutte ou de comprimé (2) restent fixés grâce à leur caractéristique adhésive, jusqu'à ce qu'ils soient retirés pour être utilisés en combinaison avec les électrodes,
dans lequel ladite électrode est une électrode cupule.

2. Système selon la revendication 1, dans lequel ledit élément de goutte ou de comprimé (2) a une taille telle qu'il est logé sur la surface d'électrode tournée vers le cuir chevelu.

3. Système selon une ou plusieurs des revendications précédentes, dans lequel des moyens de retenue sont prévus pour retenir l'électrode (1) en position sur le cuir chevelu du patient (3).

4. Système selon la revendication 3, dans lequel lesdits moyens de rétention consistent en un support adhésif.

5. Procédé de détection de signaux encéphalographiques qui implique l'utilisation du système selon une ou plusieurs des revendications 1 à 4, lequel procédé comprend la fixation de l'au moins une électrode (1) sur le cuir chevelu d'un patient (3) pour la transmission des signaux électriques détectés,
dans lequel ladite fixation implique les étapes suivantes :
a) positionner l'élément de goutte ou de comprimé (2) en contact avec ladite électrode (1),
b) lier ladite électrode (1) au cuir chevelu du patient (3) à travers ledit élément de goutte ou de comprimé (2),
c) détecter des signaux électroencéphalographiques à travers ledit élément de goutte ou de comprimé (2).

6. Procédé selon la revendication 5, dans lequel l'étape b) comprend une étape consistant à retenir lesdites électrodes (1) en position sur le cuir chevelu du patient (3) à l'aide d'un support adhésif.

7. Procédé de création des moyens de conduction et de fixation du système selon les revendications 1 à 4,
dans lequel le procédé implique les étapes suivantes :
- création de la pluralité d'éléments de goutte ou de comprimé d'hydrogel (2) selon la revendication 1,
- agencement de ladite pluralité d'éléments de goutte ou de comprimé (2) sous forme de gouttes d'hydrogel sur la surface supérieure de l'élément en forme de plaque (21) selon la revendication 1.
